(19) Europäisches Patentamt / European Patent Office / Office européen des brevets

(11) **EP 4 169 941 A1**

(12) **EUROPEAN PATENT APPLICATION**
published in accordance with Art. 153(4) EPC

(43) Date of publication:
**26.04.2023 Bulletin 2023/17**

(21) Application number: **21829606.9**

(22) Date of filing: **22.06.2021**

(51) International Patent Classification (IPC):
***C07K 16/18*** (2006.01)   ***G01N 33/574*** (2006.01)
***G01N 33/70*** (2006.01)

(52) Cooperative Patent Classification (CPC):
**C07K 16/18; G01N 33/574; G01N 33/70**

(86) International application number:
**PCT/JP2021/023578**

(87) International publication number:
**WO 2021/261483 (30.12.2021 Gazette 2021/52)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **23.06.2020 JP 2020108265**

(71) Applicants:
• **Mitsui Chemicals, Inc.**
  **Tokyo 105-7122 (JP)**
• **OSAKA UNIVERSITY**
  **Suita-shi**
  **Osaka 565-0871 (JP)**
• **University of Miyazaki**
  **Miyazaki-shi**
  **Miyazaki 889-2192 (JP)**

(72) Inventors:
• **ISHIKAWA, Komako**
  **Mobara-shi, Chiba 297-0017 (JP)**

• **AMANO, Koh**
  **Mobara-shi, Chiba 297-0017 (JP)**
• **TAKAO, Toshifumi**
  **Suita-shi, Osaka 565-0871 (JP)**
• **OKUMURA, Nobuaki**
  **Suita-shi, Osaka 565-0871 (JP)**
• **TAKEI, Toshiki**
  **Suita-shi, Osaka 565-0871 (JP)**
• **NAKAZATO, Masamitsu**
  **Miyazaki-shi, Miyazaki 889-1692 (JP)**
• **TSUBOUCHI, Hironobu**
  **Miyazaki-shi, Miyazaki 889-1692 (JP)**

(74) Representative: **Mewburn Ellis LLP**
  **Aurora Building**
  **Counterslip**
  **Bristol BS1 6BX (GB)**

Remarks:
The complete document including Reference
Table(s) and the Sequence Listing(s) can be
downloaded from the EPO website

(54) **ADENOCARCINOMA DETECTION METHOD, AND EXAMINATION KIT**

(57)   An ELISA method having an excellent high throughput property, wherein adenocarcinoma is efficiently detected with high sensitivity and specificity. An adenocarcinoma detection method based on a protein fragment of WFDC2 protein in a sample originating from a subject, the method comprising determining a presence of adenocarcinoma by comparing a first determination value and a threshold value set in advance, the first determination value being a value derived by dividing a first fragment quantity, which is a quantity of a protein fragment having an amino acid sequence of SEQ ID NO: 1 in the sample as determined by an ELISA method, by a reference quantity defined by a total quantity of WFDC2 protein or a creatinine concentration in the sample as determined by an ELISA method.

FIG.1

STANDARD PEPTIDE 1   STANDARD PEPTIDE 2   STANDARD PEPTIDE 4

EP 4 169 941 A1

**Description**

Technical Field

The present invention relates to an adenocarcinoma detection method and an examination kit.

Background Art

Adenocarcinoma is a type of epithelial malignant tumor arising from cells of secretory gland tissues. Adenocarcinoma can be generated in any organs in a body and includes lung adenocarcinoma, liver adenocarcinoma, pancreatic adenocarcinoma, lymphatic cancer, uterine adenocarcinoma, adenocarcinoma of the seminal vesicle, and gastric adenocarcinoma, or the like.

Among these, lung adenocarcinoma is a type of adenocarcinoma for which early detection is difficult, since early symptoms hardly occur. Lung adenocarcinoma occur at the highest frequency among tissue types of lung cancer, and lung adenocarcinomas account for about 40% of lung cancer cases in males, about 70% of lung cancer cases in females, and about 50% of the total lung cancer cases. Among malignant tumors, lung cancer is the number one cause of deaths in Japan. Thus, a technology for early detection of lung adenocarcinoma is required.

The survival rate for lung cancer declines with advancing of clinical stages. For example, the five-year survival rates for operable clinical stages IA, IB, IIA, IIB, and IIIA of non-small cell lung cancer are 82.0%, 66.1%, 54.5%, 46.1%, and 42.8%, respectively (Sawabata, N., et al. (2011): "Japanese lung cancer registry study of 11,663 surgical cases in 2004: demographic and prognosis changes over decade", J. Thorac. Oncol., 6(7), p. 1229-1235), and median survival times for inoperable clinical stages IIIB and IV of non-small cell lung cancer are 22.4 months (Atagi, S., et al. (2012): "Thoracic radiotherapy with or without daily low-dose carboplatin in elderly patients with non-small-cell lung cancer: a randamised, controlled, phase 3 trial by the Japan Clinical Oncology Group (JCOG0301)", Lancet Oncol., 13, p. 671-678) and 10.3 months (Scagliotti, G.V, et al. (2008): "Phase III study comparing cisplatin plus gemcitabine with cisplatin plus pemetrexed in chemotherapy-naive patients with advanced-stage non-small-cell lung cancer", J. Clin. Oncol., 26(21), p. 3543-3551), respectively.

Serum carcinoembryonic antigen (CEA), an existing tumor marker for lung adenocarcinoma, is used in clinical diagnosis. However, positive rates for CEA in lung adenocarcinoma cases are only from 36.6 to 56.5% (Molina, R., et al. (2016): "Assessment of a Combined Panel of Six Serum Tumor Markers for Lung Cancer", Am. J. Respir. Crit. Care Med., 193(4), doi.org/10.1164/rccm.201404-0603OC, and Matsuoka, K., et al. (2007): "Prognostic value of carcinoembryonic antigen and CYFRA21-1 in patients with pathological stage I non-small cell lung cancer", Eur. J. Cardiothorac. Surg., 32(3), p. 435-439), and the positive rates are particularly as low as 27% in early lung adenocarcinoma of stage I (aforementioned Matsuoka, K., et al.). Therefore, lung adenocarcinoma is difficult to diagnose using CEA at a curable stage.

As an examination method under development for clinical application, a method of detecting the presence of adenocarcinoma by detecting the presence of abnormal cleavage in a protein has been suggested (WO 2017/142025 A1). Specifically, a method of obtaining an indicator referred to as a protein fragmentation rate (Fn) by comparing a peak strength of a specific fragment of a specific protein with a peak strength of another specific fragment, in which the peak strengths are measured by multiple reaction monitoring (MRM), is described.

SUMMARY OF INVENTION

Technical Problem

As a result of examination by the present inventors, the method of obtaining a protein fragmentation rate (Fn) by an MRM method specified in the invention described in Patent Literature 1 was found to be problematic in that a sample is subjected to pretreatment, which generally takes 2 or 3 days, before being provided for the measurement, manual work is frequently required, whereby errors are likely to occur, there is a drawback concerning a property enabling multiple specimens to be simultaneously and rapidly examined (hereinafter, referred to as a "high throughput property"), and the method is difficult to apply in clinical practice.

Thus, the inventors attempted to detect lung adenocarcinoma by detecting a protein fragment characteristic of lung adenocarcinoma by a sandwich method, in order to improve the high throughput property.

However, it was found that, even though a simple change of the MRM method described in Patent Literature 1 to a sandwich method was attempted, there were cases in which the invention was not substantiated with a sandwich method, and immediate implementation of the method was difficult. For example, a fragmentation rate is obtained from a peak strength of a middle sequence and a peak strength of a specific sequence in Patent Literature 1. However, the peak strength of the specific sequence is difficult to obtain by a sandwich method. Although the inventors conducted diligent study to apply a sandwich method, a receiver operating characteristic (ROC)-area under the curve (AUC) value described in Patent Literature 1 was not obtained.

Thus, an object of an embodiment of the present invention is to provide a method and a kit by which adenocarcinoma is efficiently detected with high sensitivity and specificity by a sandwich method having an excellent high throughput property.

Solution to Problem

The solution to the problem includes the following aspects.

<1> An adenocarcinoma detection method based on a protein fragment of WFDC2 protein in a sample originating from a subject, the method including determining a presence of adenocarcinoma by comparing a first determination value and a threshold value set in advance, the first determination value being a value derived by dividing a first fragment quantity, which is a quantity of a protein fragment having an amino acid sequence of SEQ ID NO: 1 in the sample as determined by a sandwich method, by a reference quantity defined by a total quantity of WFDC2 protein or a creatinine concentration in the sample as determined by a sandwich method.

<2> The adenocarcinoma detection method according to <1>, in which a quantity of a protein fragment measured using a first anti-C-terminus antibody which specifically binds to a C-terminal region of the protein fragment having the amino acid sequence of SEQ ID NO: 1 and an anti-N-terminal side region antibody which recognizes a region nearer to an N-terminal side from the C-terminal region of the protein fragment having the amino acid sequence of SEQ ID NO: 1 is used as the first fragment quantity in the sample.

<3> The adenocarcinoma detection method according to <1> or <2>, in which a corrected determination value is used instead of the first determination value, the corrected determination value being a sum of a second determination value, which is a value derived by dividing a second fragment quantity that is the quantity of a protein fragment having an amino acid sequence of SEQ ID NO: 2 in the sample as determined by a sandwich method by the reference quantity, and a value obtained by multiplying the first determination value by from 5 to 100.

<4> The adenocarcinoma detection method according to <3> citing <2>, in which a quantity of a protein fragment measured using a second anti-C-terminus antibody which specifically binds to a C-terminal region of the protein fragment having the amino acid sequence of SEQ ID NO: 2 and an anti-N-terminal side region antibody which recognizes a region nearer to an N-terminal side from the C-terminal region of the protein fragment having the amino acid sequence of SEQ ID NO: 2 is used as the second fragment quantity in the sample.

<5> The adenocarcinoma detection method according to <2> or <4>, in which a quantity of a protein fragment measured using a third anti-C-terminus antibody which specifically binds to a C-terminal region of a protein fragment having an amino acid sequence of SEQ ID NO: 3 and an anti-N-terminal side region antibody which recognizes a region nearer to an N-terminal side from the C-terminal region of the protein fragment having the amino acid sequence of SEQ ID NO: 3 is used as the total quantity of WFDC2 protein.

<6> The adenocarcinoma detection method according to any one of <1> to <5>, in which the reference quantity is the total quantity of WFDC2 protein.

<7> The adenocarcinoma detection method according to any one of <1> to <4>, in which the reference quantity is the creatinine concentration.

<8> The adenocarcinoma detection method according to any one of <1> to <7>, in which the sample is urine of the subject or a sample originating from the urine.

<9> An examination kit for determining a presence of adenocarcinoma by a sandwich method, the examination kit including a first microplate and two types of antibodies which specifically bind to a protein fragment having an amino acid sequence of SEQ ID NO: 1, in which one of the two types of antibodies is immobilized on the first microplate.

<10> The examination kit according to <9>, in which the two types of antibodies are a first anti-C-terminus antibody which specifically binds to a C-terminal region of the protein fragment having the amino acid sequence of SEQ ID NO: 1 and an anti-N-terminal side region antibody which recognizes a region nearer to an N-terminal side from the C-terminal region of the protein fragment having the amino acid sequence of SEQ ID NO: 1.

<11> The examination kit according to <10>, further including a second microplate and a second anti-C-terminus antibody which specifically binds to a C-terminal region of a protein fragment having an amino acid sequence of SEQ ID NO: 2, in which one of the second anti-C-terminus antibody or the anti-N-terminal side region antibody is immobilized on the second microplate.

Advantageous Effects of Invention

Since the embodiments of the invention are configured as described above, it is possible to provide a method and a kit by which adenocarcinoma is efficiently detected with high sensitivity and specificity by a sandwich method

having an excellent high throughput property.

BRIEF DESCRIPTION OF DRAWINGS

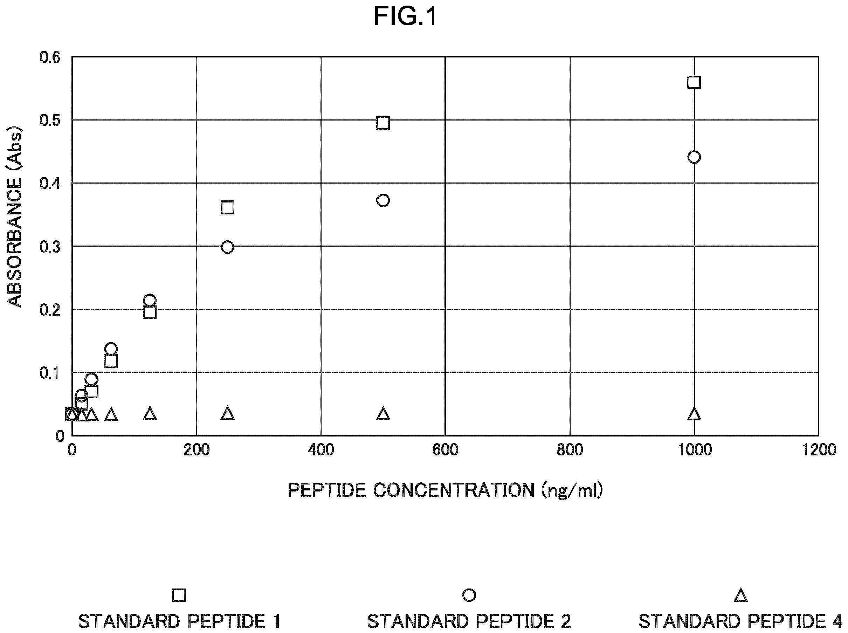

Fig. 1 is a graph obtained by plotting a correlation of concentration between absorbance and standard peptides, in a case in which ELISA was performed on a standard peptide 1, a standard peptide 2, and a standard peptide 4 by an ordinary method using a commercially available anti-HE4 antibody (Product No. ab200828, Abcam plc.) (anti-N-terminal side region antibody).

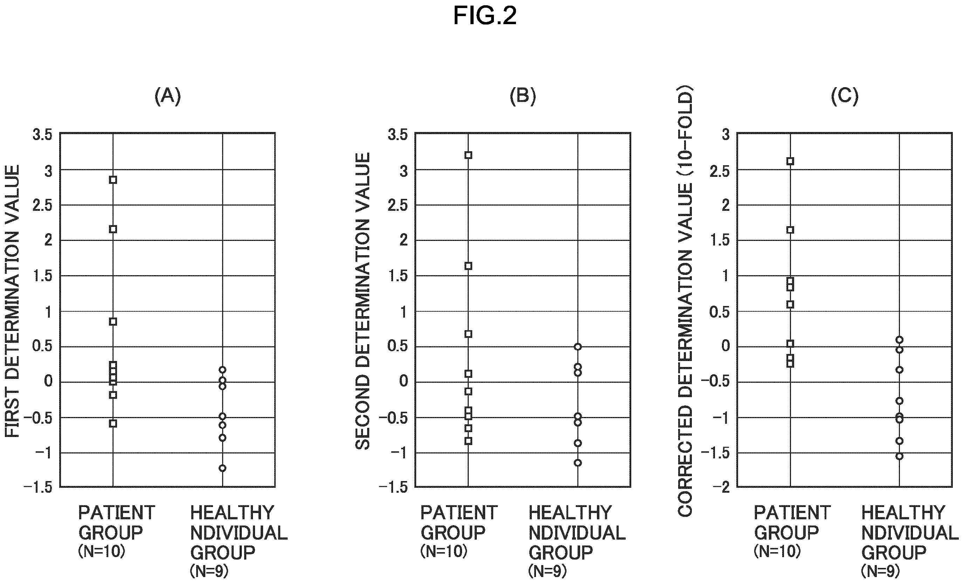

Fig. 2 is a graph obtained by plotting normalized values of each of first determination values (A), second determination values (B), and corrected determination values (C) for each of a patient group and a healthy individual group, in a case in which a creatinine concentration was used as a reference quantity.

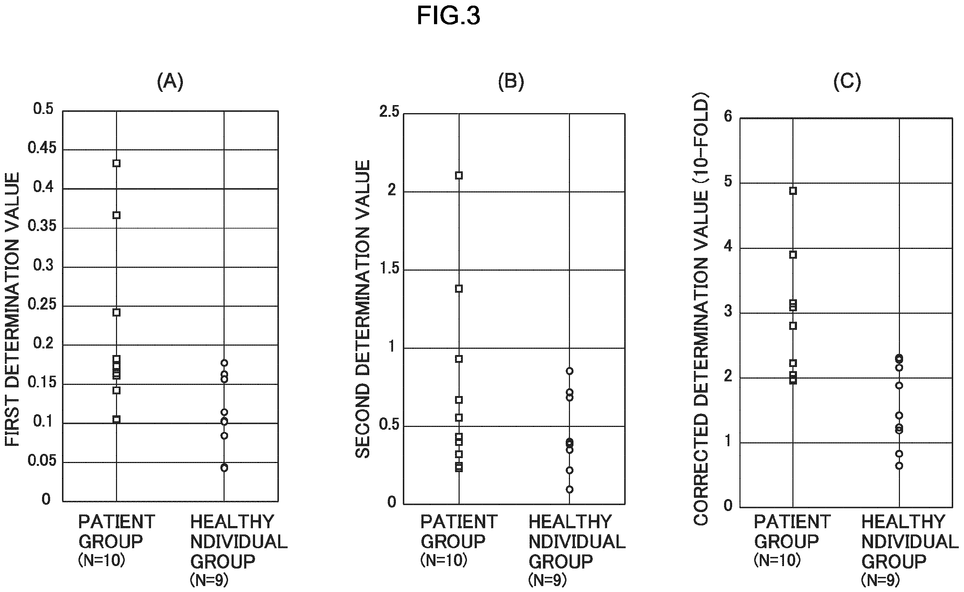

Fig. 3 is a graph obtained by plotting non-normalized values of each of the first determination values (A), the second determination values (B), and the corrected determination values (C) for each of the patient group and the healthy individual group, in a case in which the creatinine concentration was used as the reference quantity.

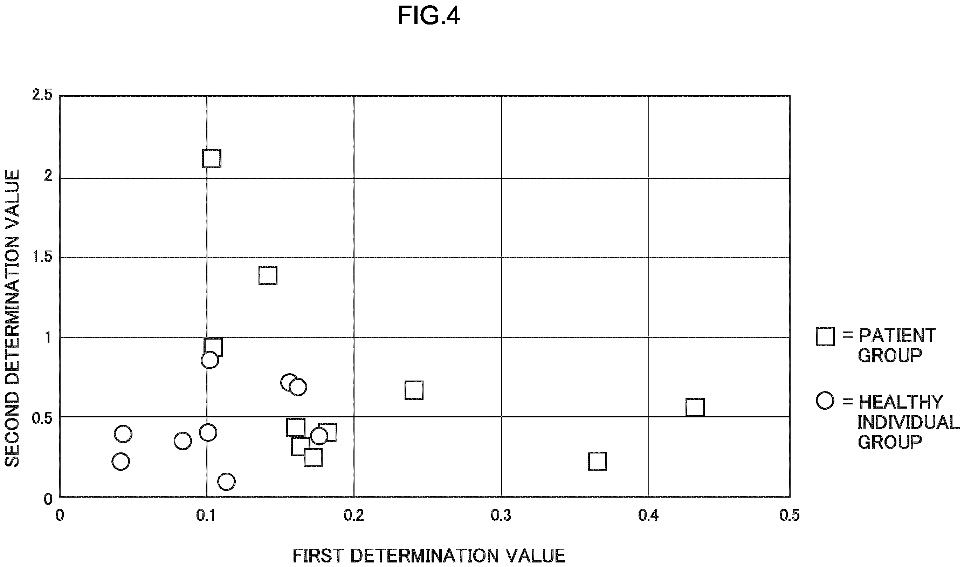

Fig. 4 is a graph obtained by two-dimensionally plotting the non-normalized second determination values against the non-normalized first determination values, in a case in which the creatinine concentration was used as the reference quantity.

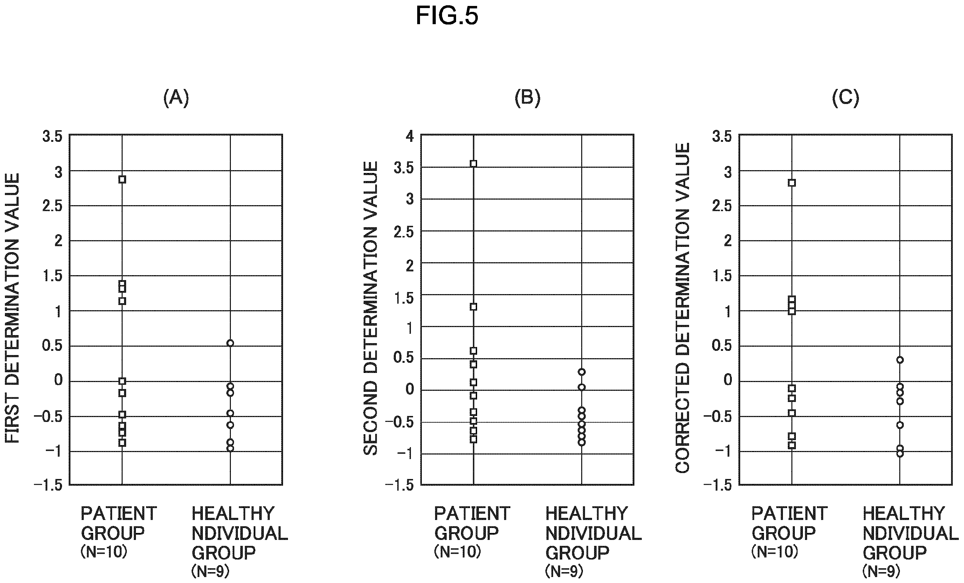

Fig. 5 is a graph obtained by plotting normalized values of each of first determination values (A), second determination values (B), and corrected determination values (C) for each of the patient group and the healthy individual group, in a case in which a total quantity of WFDC2 protein was used as a reference quantity.

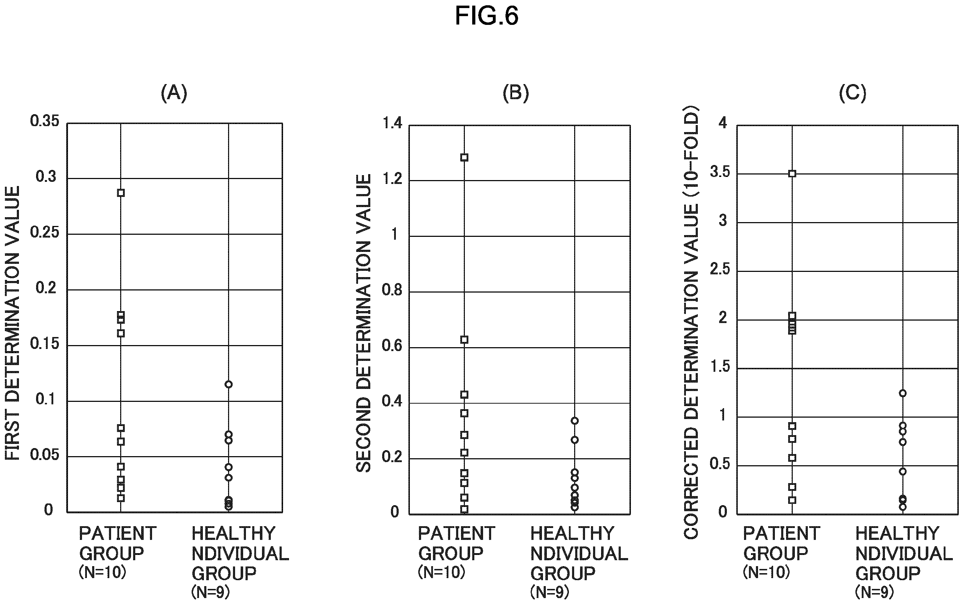

Fig. 6 is a graph obtained by plotting non-normalized values of each of the first determination values (A), the second determination values (B), and the corrected determination values (C) for each of the patient group and the healthy individual group, in a case in which the total quantity of WFDC2 protein was used as the reference quantity.

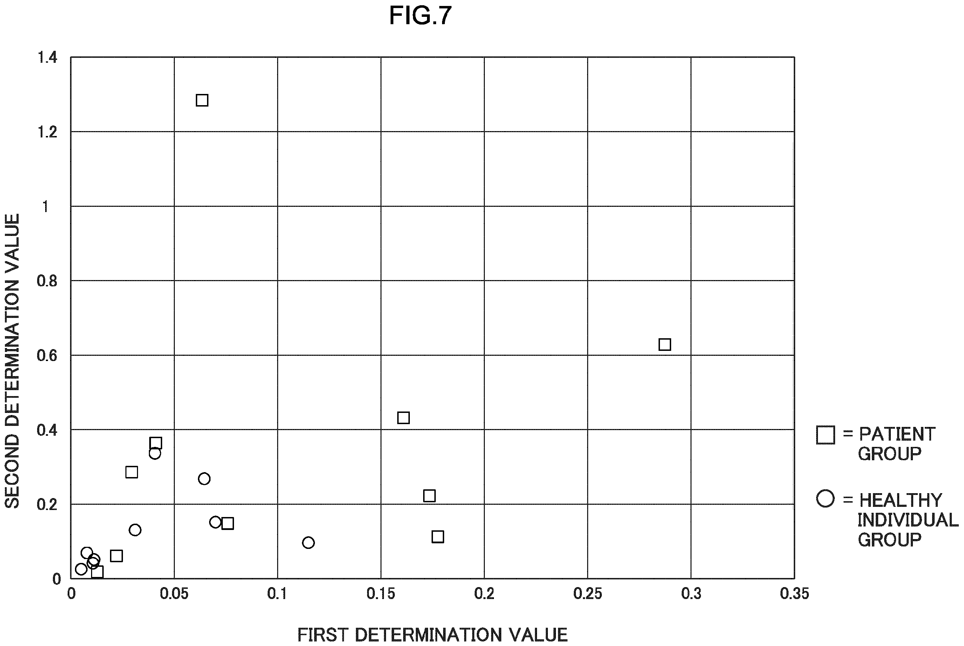

Fig. 7 is a graph obtained by two-dimensionally plotting the non-normalized second determination values against the non-normalized first determination values, in a case in which the total quantity of WFDC2 protein was used as the reference quantity.

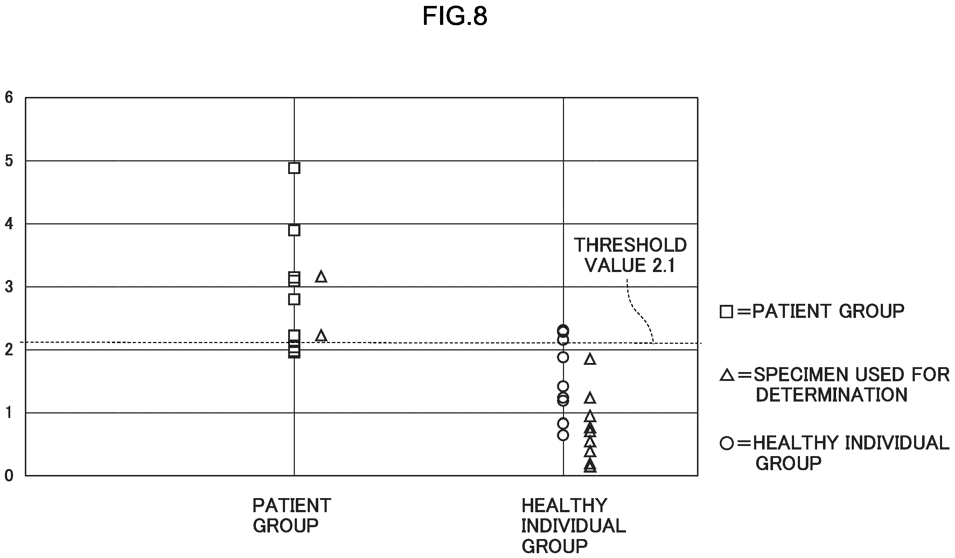

Fig. 8 is a graph obtained by plotting values obtained when performing determination on new specimens using corrected determination values for each of the patient and healthy individual groups, in a case in which a creatinine concentration was used as a reference quantity.

DESCRIPTION OF EMBODIMENTS

[First Embodiment]

An adenocarcinoma detection method according to a first embodiment is based on a protein fragment of the WFDC2 protein in a sample originating from a subject, the method including determining a presence of adenocarcinoma by comparing a first determination value and a threshold value set in advance, the first determination value being a value derived by dividing a first fragment quantity, which is the quantity of a protein fragment having an amino acid sequence of SEQ ID NO: 1 in the sample as determined by a sandwich method, by a reference quantity defined by a creatinine concentration or a total quantity of WFDC2 protein in the sample as determined by a sandwich method.

WFDC2 is the acronym for "whey acidic protein (WAP) four-disulfide core domain protein 2", and is also known as "human epididymis protein 4" (HE4). The WFDC2 protein is known to be a secretory protein of which a signal peptide at an N-terminal side is cleaved when the protein is released outside a cell. A full-length amino acid sequence of the WFDC2 protein released outside the cell typically consists of 94 amino acid residues of SEQ ID NO: 3 as shown below.
SEQ ID NO: 3: EKTGVCPELQ ADQNCTQECV SDSECADNLK CCSAGCATFC SLPNDKEGSC PQVNINFPQL GLCRDQCQVD SQCPGQMKCC RNGCGKVSCV TPNF

It has been found that a frequency of a mutation that inserts a nick between the 61st glycine (G) and the 62nd leucine (L) in the full-length amino acid sequence is higher in the WFDC2 protein of lung adenocarcinoma patients than that in the WFDC2 protein of healthy individuals. An amino acid sequence of a peptide fragment on the N-terminal side of the nick is as shown in the following SEQ ID NO: 1. Hereinafter, the peptide fragment is referred to as a "W 10409 fragment".
SEQ ID NO: 1: EKTGVCPELQ ADQNCTQECV SDSECADNLK CCSAGCATFC SLPNDKEGSC PQVNINFPQL G

In the present embodiment, one of the total quantity of WFDC2 protein or the creatinine concentration in the sample originating from the subject is measured by a sandwich method and designated as the reference quantity. The quantity of the W10409 fragment is measured by a sandwich method and designated as the first fragment quantity. The first determination value which is a value derived by dividing the first fragment quantity by the reference quantity, that is, a ratio of the W 10409 fragment to the reference quantity, is calculated, and the presence of adenocarcinoma is determined using the first determination value. The first determination value is compared with a threshold value set in advance, and in a case in which the first determination value is, for example, larger than the threshold value, it is determined that the subject is positive for adenocarcinoma.

An arbitrary value which enables a sharp distinction between the positivity and negativity for adenocarcinoma can be selected as the threshold value. For example, the first determination values described above are calculated for samples collected from plural patients known to have adenocarcinoma (for example, lung adenocarcinoma patients) in advance, and the first determination values are also calculated in the same manner for samples collected from plural healthy individuals (that is, individuals confirmed not to have adenocarcinoma). The first determination values of the adenocarcinoma patients and the healthy individuals are compared with each other, and a value that results in higher sensitivity (that is, a percentage of samples from adenocarcinoma patients that can be determined positive) and specificity (that is, a percentage of samples from healthy individuals that can be determined negative) can be used as the threshold value. For such selection of the threshold value, for example, it is conceivable to select a value that minimizes a mean square error (MSE) with reference to a Receiver Operating Characteristic-Area Under the Curve (ROC-AUC) values.

The threshold value may also be a normalized value. In a case in which a normalized value is used as the threshold value, the first determination values originating from the samples are also normalized in the same manner as the threshold value, and the normalized threshold value and the normalized first determination values are compared with each other. In the present disclosure, the term "threshold value" is a concept that includes both a non-normalized threshold value and a normalized threshold value.

Examples of the normalization include, but are not limited to, a method of calculating the normalized value n using the formula

$$n = (x - \mu)/\sigma,$$

where x is an individual value before the normalization, $\mu$ is a mean value, and $\sigma$ is a standard deviation. According to such a normalization method, a mean value and a variance for the combined group of the patient group and the healthy individual group will be 0 and 1, respectively.

By comparing the threshold value and the first determination value in this manner, the quantity of the W10409 fragment in a sample can be used as an indicator for determining the presence of adenocarcinoma with sensitivity and specificity.

A sandwich method is a method of immobilizing in advance an antibody (hereinafter referred to as a "primary antibody") against a substance to be detected (antigen) on a measurement area of a microplate, magnetic particles, a sensor chip, or the like and, after capturing a substance to be detected by immunoreaction, allowing another antibody (hereinafter referred to as a "secondary antibody") which specifically binds to the substance to be detected to bind to the substance to be detected, whereby an abundance of the antigen is measured using the secondary antibody. The method of measuring the abundance of the antigen using the secondary antibody is not particularly limited, and may be a method of measuring the abundance of the antigen using a label attached to the secondary antibody in advance or a method of measuring the abundance of the antigen using a label attached in advance to an additional antibody (hereinafter referred to as a "tertiary antibody") which specifically binds to the secondary antibody.

The primary antibody and the secondary antibody are not particularly limited as long as the object of the present embodiment can be achieved, and various antibodies described later or commercially available antibodies can be used.

The label is not particularly limited as long as the object of the present embodiment can be achieved, and a known label can be used. Examples of the label include a fluorescent dye, fluorescent nanoparticles, aggregated nanoparticles, magnetic beads, an enzyme/coenzyme, a chemiluminescent substance, and a radioactive substance. The quantity of the antigen can be measured by measuring an amount of luminescence, absorbance, fluorescence, or radiation generated from the label per se or resulting from an enzyme-substrate reaction or a chemical reaction caused by the label.

In the case of using an enzyme/coenzyme as the label, examples of the enzyme/coenzyme include a peroxidase derived from horseradish (HRP) and alkaline phosphatase (ALP). The protein quantity and the fragment quantity can be quantified by using the enzyme/coenzyme in combination with a substrate that is appropriate for the enzyme/coenzyme.

In a quantification of the protein quantity and the fragment quantity by a sandwich method, a standard curved is created using signal strengths obtained from a specimen, which is a substance originating from a living body of which a concentration is known, and the concentration of the substance to be detected can be obtained by applying signal strengths obtained from the substance to be detected.

As for the sandwich method, known methods have been in practical use, and any of the methods may be used. Specific examples of the sandwich method include enzyme-linked immunosorbent assay (ELISA), chemiluminescent immunoassay (CLIA), electro chemiluminescence immunoassay (ECLIA), and the like. Among the methods, ELISA is preferable as the sandwich method.

These sandwich methods may be performed according to standard methods. Taking ELISA as an example, specifically, a primary antibody is immobilized on wells of a microplate, a sample is added thereto, and a specific fragment or the like in the sample is allowed to bind to the primary antibody. Next, a secondary antibody is allowed to bind to the fragment, a substrate is caused to react with an enzyme for detection, such as peroxidase or the like, bound to the secondary antibody (may be allowed to bind to the secondary antibody later), and development of color, luminescence, fluorescence, or the like resulting from the reaction is measured.

The sandwich method has an excellent high throughput property, since the determination can be generally performed for from about 2 to 8 hours.

Among these sandwich methods, ELISA is preferable from a viewpoint of further improving quantitative performance, whereby ROC-AUC is further improved.

[Second Embodiment] An adenocarcinoma detection method of a second embodiment is a method in which, in the first embodiment, the quantity of a protein fragment measured using a first anti-C-terminus antibody which specifically binds to a C-terminal region of the protein fragment having an amino acid sequence of SEQ ID NO: 1 and an anti-N-terminal side region antibody which recognizes a region nearer to the N-terminal side from the C-terminal region of the protein fragment having the amino acid sequence of SEQ ID NO: 1 is used as the first fragment quantity in the sample.

The first anti-C-terminus antibody is an antibody which specifically binds to the C-terminus of the W10409 fragment. For example, an antibody which specifically binds to four C-terminal amino acid residues in the SEQ ID NO: 1, that is, "PQLG" (proline-glutamine-lysine-glycine) at the C-terminus, can be used as the first anti-C-terminus antibody. A number of amino acid residues recognized by the first anti-C-terminus antibody is not particularly limited as long as the first anti-C-terminus antibody can specifically bind to the C-terminus, and the number of the amino acid residues can be, for example, from 3 to 10.

The anti-N-terminal side region antibody is an antibody which recognizes a region nearer to the N-terminal side from the C-terminal region to which the first anti-C-terminus antibody binds. Here, the site recognized by the anti-N-terminal side region antibody may be plural consecutive amino acid residues or a tertiary structure formed by non-consecutive amino acid residues at a region nearer to the N-terminal side from the C-terminal region to which the first anti-C-terminus antibody binds. In a case in which the first anti-C-terminus antibody is, for example, the antibody which specifically binds to "PQLG" described above, the anti-N-terminal side region antibody is desirably a part of the site that recognizes an amino acid residue located at a region nearer to the N-terminal side from at least "P" (proline) of "PQLG". Note that, since it is considered that there is a competition for binding between the first anti-C-terminus antibody and the anti-N-terminal side region antibody in a case in which the recognition sites in the antigen are close to each other, it is desirable that the recognition sites in the antigen for the antibodies are located as far away from each other as possible.

With such a sandwich method in which the anti-N-terminal side region antibody and the first anti-C-terminus antibody are used, the W10409 fragment can be detected with high sensitivity in the above-described first embodiment. In the present embodiment, a fragment in which an amino acid residue is deleted at the region nearer to the N-terminal side from the site recognized by the anti-N-terminal side region antibody can also be detected in addition to the W10409 fragment, as long as the first anti-C-terminus antibody can bind to the fragment.

[Third Embodiment]

An adenocarcinoma detection method of a third embodiment is a method in which, in the first embodiment or the second embodiment, a corrected determination value is used instead of the first determination value, a corrected determination value being a sum of a second determination value, which is a value derived by dividing a second fragment quantity that is a quantity of a protein fragment having an amino acid sequence of SEQ ID NO: 2 in the sample as determined by a sandwich method by the reference quantity, and a value obtained by multiplying the first determination value by from 5 to 100.

It has been found that the frequency of a mutation that inserts a nick between the 56th asparagine (N) and the 57th phenylalanine (F) in a full-length amino acid sequence of SEQ ID NO: 3 described above is higher in the WFDC2 protein of lung adenocarcinoma patients than in the WFDC2 protein of healthy individuals, although the frequency is not as high as the frequency of the mutation that inserts a nick between the 61 st glycine (G) and the 62nd leucine (L) as described above. An amino acid sequence of a peptide fragment on the N-terminal side of the nick is as shown in a following SEQ ID NO: 2. Hereinafter, the peptide fragment is referred to as a "W14309 fragment".
SEQ ID NO: 2: EKTGVCPELQ ADQNCTQECV SDSECADNLK CCSAGCATFC SLPNDKEGSC PQVNIN

As in the first embodiment, the first fragment quantity in the present embodiment, which is the quantity of the W10409 fragment in the sample, is measured by a sandwich method, and the first determination value is calculated by dividing the first fragment quantity by the reference quantity. The second fragment quantity, which is the quantity of the W14309 fragment in the same sample, is measured by a sandwich method, and the second determination value is calculated by dividing the second fragment quantity by the reference quantity. The corrected determination value is a sum of a value obtained by multiplying the first fragment quantity by a predetermined factor, specifically, from 5 to 100, and the second determination value. In the present embodiment, the presence of adenocarcinoma is determined by using the corrected determination value. The first determination value is compared with the threshold value set in advance, and in a case in which the first determination value is, for example, larger than the threshold value, it is determined that the subject is positive for adenocarcinoma. The above-described factor is preferably from 7 to 50, more preferably from 8 to 40, and particularly preferably from 10 to 30, from the viewpoint of improving the balance between ROC-AUC and MSE.

As in the first embodiment, an arbitrary value which enables a sharp distinction between the positivity and negativity for adenocarcinoma can be selected as the threshold value, and it is desirable that the threshold value is selected while taking the second determination value into consideration as well as the first determination value. For example, the first determination values and the second determination values are calculated for samples collected from plural patients known to have adenocarcinoma (for example, lung adenocarcinoma patients) in advance to calculate the corrected determination values in the same manner as described above, and the first determination values and the second determination values are also calculated in the same manner for samples collected from plural healthy individuals (that is, individuals confirmed not to have adenocarcinoma) to calculate the corrected determination values in the same manner as described above. The corrected determination values of the adenocarcinoma patients and the healthy individuals are compared with each other, and the value that results in higher sensitivity (that is, the percentage of samples from adenocarcinoma patients that can be determined positive) and specificity (that is, the percentage of samples from healthy individuals that can be determined negative) can be used as the threshold value. For such selection of threshold value, for example, it is conceivable to select a value that minimizes a mean square error (MSE) with reference to ROC-AUC values.

The corrected determination value and the threshold value are also preferably derived by adjusting the above-described factor, multiplying the first fragment quantity by the adjusted factor, and adding the second fragment quantity to the obtained product, so as to minimize MSE. Furthermore, the corrected determination value and the threshold value are also preferably derived by adjusting the above-described factor, multiplying the first fragment quantity by the adjusted factor, and adding the second fragment quantity to the obtained product, so as to maximize ROC-AUC. Furthermore, the corrected determination value and the threshold value are also preferably derived by adjusting the above-described factor with consideration for the balance between MSE and ROC-AUC, multiplying the first fragment quantity by the adjusted factor, and adding the second fragment quantity to the obtained product.

By comparing the threshold value and the corrected determination value in this manner, the quantity of the W 10409 fragment in a sample can be used in combination with the quantity of the W14309 fragment as an indicator, and the presence of adenocarcinoma can be determined with higher sensitivity and specificity.

[Fourth Embodiment]

An adenocarcinoma detection method of a fourth embodiment is a method in which, in the third embodiment based on the second embodiment, the quantity of a protein fragment measured using a second anti-C-terminus antibody which specifically binds to a C-terminal region of the protein fragment having the amino acid sequence of SEQ ID NO: 2 and an anti-N-terminal side region antibody which recognizes a region nearer to an N-terminal side from the C-terminal region of the protein fragment having the amino acid sequence of SEQ ID NO: 2 is used as the second fragment quantity in the sample.

The second anti-C-terminus antibody is an antibody which specifically binds to the C-terminus of the W14309 fragment. For example, an antibody which specifically binds to the five C-terminal amino acid residues in the SEQ ID NO: 2, that is, "QVNIN" (glutamine-valine-asparagine-isoleucine-asparagine) at the C-terminus, can be used as the second anti-C-terminus antibody. A number of amino acid residues recognized by the second anti-C-terminus antibody is not particularly limited as long as the second anti-C-terminus antibody can specifically bind to the C-terminus, and the number of the amino acid residues can be, for example, from 3 to 10.

The anti-N-terminal side region antibody is an antibody which recognizes a region nearer to an N-terminal side from the C-terminal region to which the second anti-C-terminus antibody binds. Here, the site recognized by the anti-N-terminal side region antibody may be plural consecutive amino acid residues or a tertiary structure formed by non-consecutive amino acid residues at a region nearer to an N-terminal side from the C-terminal region to which the second anti-C-terminus antibody binds. In a case in which the second anti-C-terminus antibody is, for example, the antibody which specifically binds to "QVNIN" described above, the anti-N-terminal side region antibody is desirably a part of the site that recognizes an amino acid residue located at a region nearer to an N-terminal side from at least "Q" (glutamine) of "QVNIN". Note that, since it is considered that there is a competition for binding between the second anti-C-terminus antibody and the anti-N-terminal side region antibody in a case in which the recognition sites in the antigen are close to each other, it is desirable that the recognition sites in the antigen for the antibodies are located as far away from each other as possible.

The anti-N-terminal side region antibody for detecting the W14309 fragment may be different from the anti-N-terminal side region antibody for detecting the W10409 fragment. However, it is desirable that the anti-N-terminal side region antibodies are the same antibodies.

With such a sandwich method in which the anti-N-terminal side region antibody and the second anti-C-terminus antibody are used, the W14309 fragment can be detected with high sensitivity in the above-described third embodiment. In the present embodiment, a fragment in which an amino acid residue is deleted at a region nearer to an N-terminal side from the site recognized by the anti-N-terminal side region antibody can also be detected in addition to the W14309 fragment, as long as the second anti-C-terminus antibody can bind to the fragment.

[Fifth Embodiment]

An adenocarcinoma detection method of a fifth embodiment is a method in which, in the second embodiment or the fourth embodiment based on the third embodiment based on the second embodiment, the quantity of a protein fragment measured using a third anti-C-terminus antibody which specifically binds to a C-terminal region of a protein fragment having the amino acid sequence of SEQ ID NO: 3 and an anti-N-terminal side region antibody which recognizes a region nearer to an N-terminal side from the C-terminal region of the protein fragment having the amino acid sequence of SEQ ID NO: 3 is used as the total quantity of WFDC2 protein.

The third anti-C-terminus antibody is an antibody which specifically binds to the C-terminus of the protein fragment having the amino acid sequence of SEQ ID NO: 3. For example, an antibody which specifically binds to five C-terminal amino acid residues in the SEQ ID NO: 3, that is, "VTPNF" (valine-threonine-proline-asparagine-phenylalanine) at the C-terminus, can be used as the third anti-C-terminus antibody. A number of amino acid residues recognized by the third anti-C-terminus antibody is not particularly limited as long as the third anti-C-terminus antibody can specifically bind to the C-terminus, and the number of the amino acid residues can be, for example, from 3 to 10.

The anti-N-terminal side region antibody is an antibody which recognizes a region nearer to the N-terminal side from the C-terminal region to which the third anti-C-terminus antibody binds. Here, the site recognized by the anti-N-terminal side region antibody may be plural consecutive amino acid residues or a tertiary structure formed by non-consecutive amino acid residues at a region nearer to the N-terminal side from the C-terminal region to which the third anti-C-terminus antibody binds. In a case in which the third anti-C-terminus antibody is, for example, the antibody which specifically binds to "VTPNF" described above, the anti-N-terminal side region antibody is desirably a part of the site that recognizes an amino acid residue located at a region nearer to the N-terminal side from at least "V" (valine) of "VTPNF". Note that, since it is considered that there is a competition for binding between the third anti-C-terminus antibody and the anti-N-terminal side region antibody in a case in which the recognition sites in the antigen are close to each other, it is desirable that the recognition sites in the antigen for the antibodies are located as far away from each other as possible.

Note that the anti-N-terminal side region antibody for detecting the protein fragment having the amino acid sequence of SEQ ID NO: 3 may be different from or the same as any one of the anti-N-terminal side region antibody for detecting the W10409 fragment or the anti-N-terminal side region antibody for detecting the W14309 fragment. However, it is desirable that the anti-N-terminal side region antibody which is the same as both of the anti-N-terminal side region antibodies is used. In this case, the anti-N-terminal side region antibody is desirably at part of the site that recognizes an amino acid residue located at a region nearer to the N-terminal side from the site to which the second anti-C-terminus antibody, which binds to the C-terminus of the W14309 fragment, binds.

With such a sandwich method in which the anti-N-terminal side region antibody and the third anti-C-terminus antibody are used, the protein fragment having the amino acid sequence of SEQ ID NO: 3 can be detected with high sensitivity in the above-described second and fourth embodiments. In the present embodiment, a fragment in which an amino acid residue is deleted at a region nearer to the N-terminal side from the site recognized by the anti-N-terminal side region antibody can also be detected in addition to the protein fragment having the amino acid sequence of SEQ ID NO: 3, as long as the third anti-C-terminus antibody can bind to the fragment.

[Sixth Embodiment]

An adenocarcinoma detection method of a sixth embodiment is a method in which, in any one of the first embodiment to the fifth embodiment, the reference quantity is the total quantity of WFDC2 protein. The total quantity of WFDC2 protein include the quantities of the protein in which a nick is not inserted at any position and the protein in which a nick is inserted as described above, and the disulfide bond between the fragments separated by the nick is maintained. By using the total quantity of WFDC2 protein as the reference quantity, the quantity of a substance to be measured, the W10409 fragment or the W 143 09 fragment, in the sample can be standardized and determined as the percentage of the mutated WFDC2 protein in the total WFDC2 protein.

[Seventh Embodiment]

An adenocarcinoma detection method of a seventh embodiment is a method in which, in any one of the first embodiment to the fourth embodiment, the reference quantity is the creatinine concentration. Since creatinine is produced in an almost constant amount per day and entirely excreted in urine, creatinine is used in a clinical examination to correct an error of the concentration of the substance to be measured such as a protein or the like in urine occurring due to a difference in urine outputs. Therefore, the creatinine concentration has a significance as a reference substance in the standardization of the quantity of the substance to be measured, the W10409 fragment or the W 143 09 fragment, in the sample.

[Eighth Embodiment]

An adenocarcinoma detection method of an eighth embodiment is a method in which, in any one of the first embodiment to the seventh embodiment, the sample is urine of the subject or a sample originating from the urine. The sample is desirably the urine of the subject or a sample originating from the urine, from the viewpoints that the WFDC2 protein is excreted in urine, and the amounts of extra proteins such as serum albumin and the like are small. Here, the "sample originating from the urine" refers to urine collected from the subject and then subjected to dilution, or an appropriate reagent is added thereto for convenience of measurement.

[Ninth Embodiment]

An examination kit of a ninth embodiment is an examination kit for determining the presence of adenocarcinoma by a sandwich method, the examination kit including a first microplate and two types of antibodies which specifically bind to a protein fragment having the amino acid sequence of SEQ ID NO: 1, in which one of the two types of antibodies is immobilized on the first microplate.
A microplate consisting of a flat plate with a number of wells, each of which is used as a test tube or a petri dish, is used as the first microplate. As the microplate, a 96-well type is widely used.
The first microplate in the present embodiment contains two types of antibodies which specifically bind to the protein fragment having the amino acid sequence of SEQ ID NO: 1, and one of the two or more of antibodies is immobilized on a microplate A. The immobilization is preferably performed in all wells; however, the immobilization is not limited thereto. It is preferable that only one of the above-described two antibodies is immobilized.
The two types of antibodies included in the examination kit of the ninth embodiment are preferably antibodies that hardly compete with each other. The sites recognized by the two types of antibodies are not particularly limited as long as the amino acid sequence of SEQ ID NO: 1 can be quantified. The two types of antibodies may be polyclonal antibodies or monoclonal antibodies. The antibody that is not immobilized is included in the kit by being contained in a container. The antibody that is not immobilized may be labeled with horseradish peroxidase (HRP) or the like for quantification.
In addition to the two types of antibodies and the first microplate, the examination kit of the ninth embodiment may further include a material that is usually used in a sandwich method. Examples of the additional material that is included in the kit of the ninth embodiment can include a tertiary antibody which can specifically bind to one of the two types of antibodies, particularly, the antibody that is not immobilized, a buffer, a washing solution, a chromogenic solution, and the like. The additional material that is included in the examination kit of the ninth embodiment is not limited to these examples. The tertiary antibody may be labeled with horseradish peroxidase (HRP) or the like.
The examination kit of the ninth embodiment can be used in the adenocarcinoma detection method described in the above embodiments. Throughput can be further improved in the above-described embodiments by using the examination kit of the ninth embodiment.

[Tenth Embodiment]

An examination kit of a tenth embodiment is the kit described in the ninth embodiment, in which the two types of antibodies are a first anti-C-terminus antibody which specifically binds to a C-terminal region of the protein fragment having the amino acid sequence of SEQ ID NO: 1 and an anti-N-terminal side region antibody which recognizes a region nearer to an N-terminal side from the C-terminal region of the protein fragment having the amino acid sequence of SEQ ID NO: 1.
The first anti-C-terminus antibody and the anti-N-terminal side region antibody are the same as those in the second embodiment.

[Eleventh Embodiment]

An examination kit of an eleventh embodiment is the examination kit described in the tenth embodiment, the examination kit further including a second microplate and a second anti-C-terminus antibody which specifically binds to a C-terminal region of a protein fragment having the amino acid sequence of SEQ ID NO: 2, in which one of the second anti-C-terminus antibody or the anti-N-terminal side region antibody is immobilized on the second microplate.

One of the second anti-C-terminus antibody and the anti-N-terminal side region antibody is immobilized on the second microplate according to the present embodiment.

Details of the microplate used as the second microplate are the same as those in the ninth embodiment. The second anti-C-terminus antibody and the anti-N-terminal side region antibody are the same as those in the fourth embodiment.

[Examples]

(1) Preparation of Samples for Measurement and Standard Preparations

(1-1) Sample Collection

Among patients showing a nodule shadow or a mass shadow in the lung which is suspected to be primary lung cancer in chest imaging examination, ten patients who were diagnosed with lung adenocarcinoma by surgery, transbronchial biopsy, lymph node biopsy, or cytodiagnosis were selected. Using sterile cups, first-catch urine was collected from the the selected lung adenocarcinoma patients and nine healthy individuals as samples, and the samples were stored at -80°C until measurement. When performing the measurement, the frozen urine naturally thawed at room temperature were used as samples, and were provided for analysis of the total quantities of WFDC2 protein, the quantities of WFDC2 protein fragment, and the creatinine concentrations.

(1-2) Synthesis of Peptides to Be Used as Standard Preparations

A standard peptide 1 and a standard peptide 2 were synthesized as standard preparations corresponding to the W10409 fragment and the W14309 fragment, respectively, by an ordinary method, Fmoc solid-phase peptide synthesis. Crude peptides were obtained through a usual deprotection process using a trifluoroacetic acid (TFA) cocktail. Disulfide bonds were formed between cysteine (C) residues in the crude peptides under a redox condition obtained using glutathione, and purification was performed by reverse phase HPLC, whereby the following standard peptide 1 set forth in SEQ ID NO: 4 and the standard peptide 2 set forth in SEQ ID NO: 5 were obtained. After the purification, the purity of the standard peptide 1 was 96%, and the purity of the standard peptide 2 was 98%.

SEQ ID NO: 4 (standard peptide 1): TGAEKTGVCP ELQADQNCTQ ECVSDSECAD NLKCCSAGCA TFCSLPNDKE GSSPQVNINF PQLG

SEQ ID NO: 5 (standard peptide 2): TGAEKTGVCP ELQADQNCTQ ECVSDSECAD NLKCCSAGCA TFCSLPNDKE GSSPQVNIN

In both of the standard peptide 1 and the standard peptide 2, three amino acid residues threonine (T)-glycine (G)-alanine (A) were added to the N-termini of the corresponding W10409 fragment and the W 143 09 fragment, respectively, whereas the C-termini of the peptides are the same as those of the corresponding fragments. Furthermore, the 50th amino acid residues cysteine (C) in SEQ ID NO: 1 of the W10409 fragment and SEQ ID NO: 2 of the W14309 fragment were substituted with serine (S) in the standard peptide 1 and the standard peptide 2 at the corresponding 53rd positions. It is considered that the sulfhydryl groups of the 50th cysteine residues in SEQ ID NO: 1 and SEQ ID NO: 2 form disulfide bonds with the sulfhydryl group of the 80th cysteine residue in SEQ ID NO: 3. In the case of the absence of the 80th cysteine residue, the sulfhydryl groups in SEQ ID NO: 1 and SEQ ID NO: 2 form disulfide bonds with other cysteine residues, and the tertiary structures may be different from those of the protein fragments of SEQ ID NO: 1 and SEQ ID NO: 2 existing in the living body. Changes in the tertiary structures may result in an inability to be recognized by an antibody. For this reason, the amino acid substitution was performed as described above.

Two peptide fragments of a standard peptide 3, which was a standard preparation corresponding to the full-length WFDC2 protein, were synthesized by the same Fmoc solid-phase peptide synthesis, and the full-length sequence was constructed using an ordinary chemical protein synthesis method, native chemical ligation. A disulfide bond was formed between cysteine (C) residues under a redox condition obtained using glutathione, and purification was performed by reverse phase HPLC, whereby the following standard peptide 3 set forth in SEQ ID NO: 6 was obtained. After the purification, the purity of the standard peptide 3 was 95%. The only difference between the standard peptide 3 and the full-length WFDC2 protein set forth in SEQ ID NO: 3 is the three amino acid residues threonine (T)-glycine (G)-alanine (A) added to the N-terminus of the standard peptide 3.

SEQ ID NO: 6 (standard peptide 3): TGAEKTGVCP ELQADQNCTQ ECVSDSECAD NLKCCSAGCA TFCSLPNDKE GSCPQVNINF PQLGLCRDQC QVDSQCPGQM KCCRNGCGKV SCVTPNF

(1-3) Preparation of First Anti-C-Terminus Antibody

The first anti-C-terminus antibody was prepared as an antibody for recognizing the C-terminus of the W10409 fragment set forth in SEQ ID NO: 1, based on the method described in "New Edition Anti-Peptide Antibody Experimental Protocol" (compiled under the supervision of Ohmi Shinobu, Kunio Tsujimura, and Masaki Inagaki, Gakken Medical Shujunsha Co., Ltd., published September 6, 2004). A synthetic peptide set forth in the following SEQ ID NO: 7 was obtained by adding CGGG (cysteine-glycine-glycine-glycine) on the N-terminal side of PQLG (proline-glutamine-lysine-glycine), which was the C-terminus of the W10409 fragment, and used as the immunogen.
SEQ ID NO: 7: CGGGPQLG
The synthetic peptide was administered to a chicken by intravenous injection at weekly intervals. Antibody titer in the chicken serum was measured by ELISA after the fourth administration. As a result, sufficient increase in the antibody titer was observed. Therefore, the antiserum was collected one week after the day of the final immunization.
The synthetic peptide of SEQ ID NO: 7 was immobilized on an agarose carrier, and the antibody was purified from the antiserum using the immobilized synthetic peptide as the affinity column.
By western blotting, it was confirmed that the antibody bound to the peptide of which the terminus was PQLG, whereas the antibody did not bind to a peptide containing PQLG only in the middle of the sequence. The antibody obtained in this manner was an IgY subclass, and this antibody was used as the first anti-C-terminus antibody.

(1-4) Preparation of Second Anti-C-Terminus Antibody

The second anti-C-terminus antibody was prepared as an antibody for recognizing the C-terminus of the W14309 fragment set forth in SEQ ID NO: 2, based on the method described in "New Edition Anti-Peptide Antibody Experimental Protocol" indicated in (1-3). A synthetic peptide set forth in the following SEQ ID NO: 8 was obtained by adding CGGG on the N-terminal side of QVNIN (glutamine-valine-asparagine-isoleucine-asparagine), which was the C-terminus of the W14309 fragment, and used as the immunogen.
SEQ ID NO: 8: CGGGQVNIN
The synthetic peptide was administered to a rabbit by intravenous injection at weekly intervals. Antibody titer in the rabbit serum was measured by ELISA after the fourth administration. As a result, sufficient increase in the antibody titer was observed. Therefore, the antiserum was collected one week after the day of the final immunization.
The synthetic peptide of SEQ ID NO: 8 was immobilized on an agarose carrier, and the antibody was purified from the antiserum using the immobilized synthetic peptide as the affinity column.
By western blotting, it was confirmed that the antibody bound to the peptide of which the terminus was QVNIN, whereas the antibody did not bind to a peptide containing QVNIN only in the middle of the sequence. The antibody obtained in this manner was an IgG subclass, and this antibody was used as the second anti-C-terminus antibody.

(1-5) Preparation of Third Anti-C-Terminus Antibody

The third anti-C-terminus antibody was prepared as an antibody for recognizing the C-terminus of the full-length WFDC2 protein set forth in SEQ ID NO: 3, based on the method described in "New Edition Anti-Peptide Antibody Experimental Protocol" indicated in (1-3). A synthetic peptide set forth in the following SEQ ID NO: 9 was obtained by adding CGGG on the N-terminal side of TPNF (threonine-proline-asparagine-phenylalanine), which was the C-terminus of the full-length WFDC2 protein, and used as the immunogen.
SEQ ID NO: 9: CGGGTPNF
The synthetic peptide was administered to a chicken by intravenous injection at weekly intervals. Antibody titer in the chicken serum was measured by ELISA after the fourth administration. As a result, sufficient increase in the antibody titer was observed. Therefore, the antiserum was collected one week after the day of the final immunization.

The synthetic peptide of SEQ ID NO: 9 was immobilized on an agarose carrier, and the antibody was purified from the antiserum using the immobilized synthetic peptide as the affinity column.

By western blotting, it was confirmed that the antibody bound to the peptide of which the terminus was TPNF, whereas the antibody did not bind to a peptide containing TPNF only in the middle of the sequence. The antibody obtained in this manner was an IgY subclass, and this antibody was used as the third anti-C-terminus antibody.

(1-6) Anti-N-terminal Side Region Antibody

A commercially available anti-HE4 antibody (Product No. ab200828, Abcam plc.) was used as the anti-N-terminal side region antibody. It is presumed that this antibody recognizes an amino acid sequence or a tertiary structure formed by several amino acids on a region nearer to the N-terminal side from QVNIN, which is the amino acid sequence recognized by the second anti-C-terminus antibody, in SEQ ID NO: 1, SEQ ID NO: 2, and SEQ ID NO: 3.

In order to identify the recognition site in the antigen, a standard peptide 4 set forth in the following SEQ ID NO: 10 was synthesized as a peptide on ta region nearer to the C-terminal side from the standard peptide 1 (SEQ ID NO: 1) and the standard peptide 2 (SEQ ID NO: 2). The synthesis method for the standard peptide 4 was based on the synthesis methods for the standard peptide 1 and the standard peptide 2.

SEQ ID NO: 10 (standard peptide 4): RDQCQVDSQCPGQMKCCRNGCGKVSCVTPNF

ELISA was performed by an ordinary method on various concentrations of the standard peptide 1, the standard peptide 2, and the standard peptide 4 using the commercially available anti-HE4 antibody (Product No. ab200828, Abcam plc.) and each antibody recognizing the C-terminus, and absorbance was measured for each peptide, whereby the presence of concentration dependency was confirmed. The results are shown in Fig. 1.

As shown in Fig. 1 and as is suggested from the lack of competition with the antibodies recognizing the C-terminus in the following Example, the commercially available anti-HE4 antibody (Product No. ab200828, Abcam plc.) was found to recognize the amino acid sequence or the tertiary structure formed by several amino acids on the N-terminal side.

(2) Example 1

In Example 1, the case of using the first fragment quantity which is the W10409 fragment quantity as the indicator, the case of using the second fragment quantity which is the W14309 fragment quantity as the indicator, and the case of using the combination of the first fragment quantity and the second fragment quantity as the indicator were examined as lung adenocarcinoma detection models by using the creatinine concentration in the sample urine as the reference quantity.

(2-1) Measurement of Creatinine Concentration in Urine

The creatinine concentration in each of the obtained samples was measured using a kit for measuring creatinine in urine samples (Creatinine, Assay Kit, Colorimetric, for Urine Sample, Product No. 500701, Cayman Chemical Company). The protocol was based on the method described in the kit.

(2-2) Measurement of First Fragment Quantity

The first fragment quantity in the sample obtained from each of the lung adenocarcinoma patients and healthy individuals was measured by ELISA. The anti-N-terminal side region antibody described above was diluted with a phosphate-buffered solution (PBS) containing 1 weight% bovine serum albumin (BSA) (hereinafter, referred to as "BSA-PBS") at 1:4000, and 100 μL of the diluted anti-N-terminal side region antibody was added to each well of an ELISA plate (Nunc-Immuno (registered trademark) MicroWell (registered trademark) 96 well solid plates, Merck KGaA) as the primary antibody. The antibody was immobilized overnight at 4°C. Next, the ELISA plate was washed three times with PBS-Tween, and then 200 μL of Blocking One (Product No. 03953-66, Nacalai Tesque, Inc.) which had been diluted with pure water at 1:5 was added to each well. The plate was incubated for 2 hours at 25°C, and then washing was performed three times with PBS with Tween (hereinafter, referred to as "PBS-Tween"). The samples were diluted with BSA-PBS at 1:3, and 100 μL of the diluted samples were added to each well. The plate was incubated for 2 hours at 25°C, and washing was performed three times with PBS-Tween. The first anti-C-terminus antibody was diluted with BSA-PBS at 1:5000, and 100 μL of the diluted first anti-C-terminus antibody was added to each well as the secondary antibody. The plate was incubated for 1.5 hours at 25°C, and washing was performed three times with PBS-Tween. A horseradish peroxidase (HRP)-labeled antibody (Peroxidase Affinity pure Donkey Anti-Chicken IgY (IgG) (H+L), Product No.703-035-155, Jacson ImmunoResearch Laboratories Inc.) which is an anti-chicken IgY antibody was diluted with BSA-PBS at 1:5000, and 100 μL of the diluted HRP-labeled antibody was added to each well. The plate was incubated for 1 hour at 25°C, and washing was performed five times with PBS-Tween. Then, 100 μL of TMB Substrate Ultra Solution (Product No. ES022-100ML, Merc KGaA) was added to each well as the substrate. After from 5 to 10 minutes of the enzymatic reaction at 25°C, sulfuric acid was added to stop the reaction, and absorbance at 450 nm was measured using a microplate reader (iMark microplate reader, Bio-Rad Laboratories, Inc.). The measured absorbance was applied to a standard curve created in advance using the standard peptide 1, and the first fragment quantity was calculated.

(2-3) Measurement of Second Fragment Quantity

The second fragment quantity in the sample obtained from each of the lung adenocarcinoma patients and healthy individuals was measured by ELISA. The second anti-C-terminus antibody described above was diluted with BSA-PBS at 1:4000, and 100 μL of the diluted second anti-C-terminus antibody was added to each well of an ELISA plate (Nunc-Immuno (registered trademark) MicroWell (registered trademark) 96 well solid plates, Merck KGaA) as the primary antibody. The antibody was immobilized overnight at 4°C. Next, the ELISA plate was washed three times with PBS-Tween, and then 200 μL of Blocking One (Product No. 03953-66, Nacalai Tesque, Inc.) which had been diluted with pure water at 1:5 was added to each well. The plate was incubated for 2 hours at 25°C, and then washing was performed three times with PBS-Tween. The samples were diluted with BSA-PBS at 1:3, and 100 μL of the diluted samples were added to each well. The plate was incubated for 2 hours at 25°C, and washing was performed three times with PBS-Tween. Next, the anti-N-terminal side region antibody that had been subjected to peroxidase labeling using a peroxidase labeling kit (Dojindo Labeling Kit series Peroxidase Labeling Kit, Product No. LK11, Dojindo Molecular Technologies, Inc.) was diluted with BSA-PBS at 1:2000, and 100 μL of the diluted anti-N-terminal side region antibody was added to each well as the secondary antibody. The plate was incubated for 1.5 hours at room temperature. Then, 100 μL of TMB Substrate Ultra Solution (Product No. ES022-100ML, Merck KGaA) was added to each well as the substrate. After from 5 to 10 minutes of the enzymatic reaction at 25°C, sulfuric acid was added to stop the reaction, and absorbance at 450 nm was measured using a microplate reader (iMark microplate reader, Bio-Rad Laboratories, Inc.). The measured absorbance was applied to a standard curve created in advance using the standard peptide 2, and the second fragment quantity was calculated.

(2-4) Calculation of First Determination Value, Second Determination Value, and Corrected Determination Value

The first fragment quantity obtained from each sample was divided by the creatinine concentration in the urine, thereby obtaining the first determination value. Similarly, the second fragment quantity obtained from each sample was divided by the creatinine concentration in the urine, thereby obtaining the second determination value. For each sample, a sum of a value obtained by multiplying the first determination value by 10 and the second determination value was obtained as a corrected determination value (10-fold). In addition, the factor was changed to 20 and 30 to obtain corrected determination values (20-fold) and corrected determination values (30-fold).

The first determination values, the second determination values, and the corrected determination values obtained above were all normalized with the mean value and the standard deviation of the combined group of the patient group and the healthy individual group. Specifically, the normalized value n was calculated using the formula

$$n = (x - \mu)/\sigma,$$

where x was an individual value before the normalization, μ was the mean value, and σ was the standard deviation. Due to this normalization, the mean value and the variance for the combined group of the patient group and the healthy individual group were 0 and 1, respectively. The first determination values, the second determination values, and the corrected determination values (10-fold) normalized in this manner were plotted on the graphs shown in Fig. 2(A), Fig. 2(B), and Fig. 2(C), respectively.

The non-normalized first determination values, the non-normalized second determination values, and the non-normalized corrected determination values (10-fold) were plotted on the graphs shown in Fig. 3(A), Fig. 3(B), and Fig. 3(C), respectively.

(2-5) Determination of Threshold Value

Next, the degree of overlap between the patient group and the healthy individual group was analyzed in each of the first determination values, the second determination values, and each of the corrected determination values in both cases of performing the normalization and not performing the normalization. Here, the "degree of overlap" indicates how much the low range of each of the determination values obtained in the patient group overlaps with the high range of each of the determination values obtained in the healthy individual group. The term also indicates that the narrower the overlapping range, the sharper the determination value can distinguish between the patient group and the healthy individual group.

First, in a case in which the normalization is performed, Fig. 2(A) and Fig. 2(B) are compared with each other, as a result of which it is found at first glance that the degree of overlap in the first determination values in Fig. 2(A) is smaller than the degree of overlap in the second determination values in Fig. 2(B). The degree of overlap in the corrected determination values in Fig. 2(C) is found to be smaller than the degrees of overlap in both the first determination values in Fig. 2(A) and the second determination values in Fig. 2(B).

In a case in which the normalization is not performed, Fig. 3(A) and Fig. 3(B) are compared with each other, as a result of which it is found at first glance that the degree of overlap in the first determination values in Fig. 3(A) is smaller than the degree of overlap in the second determination values in Fig. 3(B). The degree of overlap in the corrected determination values in Fig. 3(C) is found to be smaller than the degrees of overlap in both the first determination values in Fig. 3(A) and the second determination values in Fig. 3(B).

Next, the values of ROC-AUC were calculated in a case in which the normalized first determination values, second determination values, corrected determination values (10-fold), corrected determination values (20-fold), and corrected determination values (30-fold) were used as the indicators for examination, and the calculated ROC-AUC values were 0.83, 0.62, 0.84, 0.87, and 0.83, respectively. From the ROC-AUC values, it is possible to say that the first determination values that are based on the W10409 fragment quantity are superior as the indicators for examination of lung adenocarcinoma to the second determination values that are based on the W14309 fragment quantity. It is also possible to say that the corrected determination values obtained by combining the first determination values and the second determination values are further superior as the indicators for the examination compared to the case of using each of the first determination values and the second determination values independently. The ROC-AUC values in a case in which the normalization is not performed are the same as above.

A graph obtained by two-dimensionally plotting the non-normalized second determination values against the non-normalized first determination values for each sample is shown in Fig. 4. In the graph, the circular symbols represent the samples of the healthy individuals, and the square symbols represent the samples of the patients. As can be understood from the graph, no conspicuous values are observed in any of the first determination values and the second determination values for the samples of the healthy individuals, whereas the samples of the patients tend to show conspicuous values in either the first determination values or the second determination values. In other words, it is assumed that either a mutation that results in the generation of the W10409 fragment or a mutation that results in the generation of the W14309 fragment occurs in the WFDC2 protein of the lung adenocarcinoma patients. In addition, the frequency of the mutation that results in the generation of the W10409 fragment is higher than the frequency of the mutation that results in the generation of the W14309 fragment, which can explain the superiority of the first determination values to the second determination values as the indicators for the examination. It is also presumed that, although the positivity for the mutation that results in the generation of the W14309 fragment cannot be determined with the first determination values, the positivity for the mutation can be determined by taking the second determination values into account as well, while reducing the number of misses to a certain extent.

The calculation of the threshold values for the degrees of overlap between the patient group and the healthy individual group was performed by using each of the normalized data shown in Fig. 2(A) to Fig. 2(C) and the non-normalized data shown in Fig. 3(A) to Fig. 3(C), and using MSE as the index and each of the first determination values, the second determination values, and the corrected determination values as the indicators for the examination. MSE was calculated by the following mathematical formula.

[Math 1]

$$(MSE) = \frac{1}{N} \left\{ \sum_i^{patient} (\max(0, t - x_i))^2 + \sum_j^{healthy} (\max(0, x_j - t))^2 \right\}$$

In the mathematical formula, N represents the total number of samples, t represents the threshold value, $x_i$ represents an individual determination value in the patient group, and $x_j$ represents an individual determination value in the healthy individual group. To add a supplementary explanation of the mathematical formula, only the differences between the threshold value (t) and the individual determination values in the patient group ($x_i$) that are below the threshold value (t) are squared and then added up, and only the differences between the threshold value (t) and the individual determination values in the healthy individual group ($x_j$) that are above the threshold value (t) are squared and then added up. The sum of the resulting values is divided by the total number of samples (N) to obtain the value of MSE. From the mathematical formula, it is understood that the smaller the degree of overlap between the two groups is, the smaller the MSE value is.

From the mathematical formula, the value of t that minimized MSE was designated as the threshold value for each of the determination values.

As a result, MSE for the normalized first determination values was 0.0269, which was the minimum, when the threshold value was -0.21. MSE for the normalized second determination values was 0.0948, which was the minimum, when the threshold value was 0.62. MSE for the normalized corrected determination values (10-fold) was 0.00681, which was the minimum, when the threshold value was -0.106. MSE for the normalized corrected determination values (20-fold) was 0.0835 when the threshold value was -0.079. MSE for the normalized corrected determination values (30-fold) was 0.01258 when the threshold value was 0.08405.

MSE for the non-normalized first determination values was 0.000245, which was the minimum, when the threshold value was 0.141. MSE for the non-normalized second determination values was 0.02, which was the minimum, when the threshold value was 0.485. MSE for the normalized corrected determination values (10-fold) was 0.0069, which was the minimum, when the threshold value was 2.1. MSE for the non-normalized corrected determination values (20-fold) was 0.03 when the threshold value was 3.668. MSE for the non-normalized corrected determination values (30-fold) was 0.087 when the threshold value was 5.192.

ROC-AUC, the threshold value, and MSE for each of the normalized determination values described above are indicated in the following Table 1, and ROC-AUC, the threshold value, and MSE for each of the non-normalized determination values described above are indicated in the following Table 2.

[Table 1]

| Determination value | Fragment | ROC-AUC | Threshold value | MSE |
|---|---|---|---|---|
| First determination value | W10409 | 0.83 | -0.21 | 0.0269 |
| Second determination value | W14309 | 0.62 | 0.62 | 0.0948 |
| Corrected determination value (10-fold) | W10409 + W 14309 | 0.84 | -0.106 | 0.00681 |
| Corrected determination value (20-fold) | W10409 + W 14309 | 0.87 | -0.079 | 0.0835 |
| Corrected determination value (30-fold) | W10409 + W14309 | 0.83 | 0.08405 | 0.01258 |

[Table 2]

| Determination value | Fragment | ROC-AUC | Threshold value | MSE |
|---|---|---|---|---|
| First determination value | W10409 | 0.83 | 0.141 | 0.000245 |
| Second determination value | W14309 | 0.62 | 0.485 | 0.020351 |
| Corrected determination value (10-fold) | W10409 + W14309 | 0.84 | 2.1 | 0.006905 |
| Corrected determination value (20-fold) | W10409 + W14309 | 0.87 | 3.66 | 0.030359 |
| Corrected determination value (30-fold) | W10409 + W14309 | 0.83 | 5.19 | 0.08767 |

From the above, it was found that the determination model using the first determination values based on the detection of the W10409 fragment was superior as the lung adenocarcinoma detection method compared to the determination model using the second determination values based on the detection of the W14309 fragment, since ROC-AUC was extremely higher, and MSE was smaller in the determination model using the first determination values.

In the determination model using the corrected determination values, in which the second determination values are taken into consideration as well while placing more weight on the first determination values, the ROC-AUC values were either higher than or almost equal to those in the determination model using the first determination values alone. Furthermore, the MSE values were significantly low for all corrected determination values. Therefore, it was found that the determination model using the corrected determination values was further superior as the lung adenocarcinoma detection method to the determination model using the first determination values alone.

It was also found that each of the determination values may or may not be normalized.

(2-6) Detection of Lung Adenocarcinoma

In a case in which the detection of the W10409 fragment is used alone as the indicator, the lung adenocarcinoma detection method is as follows. That is, urine is collected from a subject who has not been identified as a lung adenocarcinoma patient or a healthy subject yet, and using the urine as a sample, the creatinine concentration in the urine is measured according to (2-1), and the first fragment quantity is measured according to (2-2). The first determination value is calculated and subjected to normalization according to (2-4), and in a case in which the normalized first determination value is -0.21 indicated in Table 1 or higher, the subject can be determined to be positive for lung adenocarcinoma. In the case of not performing the normalization, the first determination value before the normalization is used for the comparison with the threshold value, and in a case in which the first determination value is 0.141 indicated in Table 2 or higher, the subject can be determined to be positive for lung adenocarcinoma.

In a case in which the detection of the W 143 09 fragment is also used in addition to the detection of the W10409 fragment, the lung adenocarcinoma detection method is as follows. That is, urine is collected from a subject who has not been identified as a lung adenocarcinoma patient or a healthy subject yet, and using the urine as a sample, the creatinine concentration in the urine is measured according to (2-1), the first fragment quantity is measured according to (2-2), and the second fragment quantity is measured according to (2-3). The first determination value and the second determination value are calculated, and the corrected determination value is calculated from these determination values and subjected to normalization according to (2-4). In a case in which the normalized corrected determination value is -0.106 indicated in Table 1 or higher, the subject can be determined to be positive for lung adenocarcinoma. In the case of not performing the normalization, the corrected determination value before the normalization is used for the comparison with the threshold value, and in a case in which the corrected determination value is 2.1 indicated in Table 2 or higher, the subject can be determined to be positive for lung adenocarcinoma. An example of using a 10-fold corrected determination value has been described herein, and the same description is applied to the corrected determination values of other factors as well. The same applies to Example 2 described below.

In the above-described detection methods, ELISA is adopted for the measurement of the first fragment quantity and the second fragment quantity, the process from the addition of the sample originating from a specimen to the obtainment of each of the determination values is completed within 8 hours, and it is possible to simultaneously measure multiple specimens within a short period of time with a general-purpose automated equipment, whereby the methods have an excellent high throughput property. The same applies to Example 2 described below.

(3) Example 2

In Example 2, the case of using the first fragment quantity which is the W10409 fragment quantity as the indicator, the case of using the second fragment quantity which is the W14309 fragment quantity as the indicator, and the case of using the combination of the first fragment quantity and the second fragment quantity as the indicator were examined as the lung adenocarcinoma detection models by using the total quantity of WFDC2 protein in the sample urine as the reference quantity.

(3-1) Measurement of Total Quantity of WFDC2 Protein

The total quantities of WFDC2 protein in the same samples as those in Example 1 were measured by ELISA. The third anti-C-terminus antibody described above was diluted with BSA-PBS at 1:8000, and 100 $\mu$L of the diluted third anti-C-terminus antibody was added to each well of an ELISA plate (Nunc-Immuno (registered trademark) MicroWell (registered trademark) 96 well solid plates, Merck KGaA) as the primary antibody. The antibody was immobilized overnight at 4°C. Next, the ELISA plate was washed three times with PBS-Tween, and then 200 $\mu$L of Blocking One (Product No. 03953-66, Nacalai Tesque, Inc.) which had been diluted with pure water at 1:5 was added to each well. The plate was incubated for 2 hours at 25°C, and then washing was performed three times with PBS-Tween. The samples were diluted with BSA-PBS at 1:3, and 100 $\mu$L of the diluted samples were added to each well. The plate was incubated for 2 hours at 25°C, and washing was performed 3 times with PBS-Tween. The anti-N-terminal side region antibody described above was diluted with BSA-PBS at 1:1000, and 100 $\mu$L of the diluted anti-N-terminal side region antibody was added to each well as the secondary antibody. The plate was incubated for 1.5 hours at 25°C, and washing was performed three times with PBS-Tween. A horseradish peroxidase (HRP)-labeled antibody (Peroxidase Affinity pure Donkey Anti-Rabbit IgG, Product No. 711-035-152, Jacson ImmunoResearch Laboratories Inc.) which recognizes the N-terminal side region antibody described above was diluted with BSA-PBS at 1:5000, and 100 $\mu$L of the diluted HRP-labeled antibody was added to each well. The plate was incubated for 1 hour at 25°C, and washing was performed five times with PBS-Tween. Then, 100 $\mu$L of TMB Substrate Ultra Solution (Product No. ES022-100ML, Merck KGaA) was added to each well as the substrate. After from 5 to 10 minutes of the enzymatic reaction at 25°C, sulfuric acid was added to stop the reaction, and absorbance at 450 nm was measured using a microplate reader (iMark microplate reader, Bio-Rad Laboratories, Inc.). The measured absorbance was applied to a standard curve created in advance using the standard peptide 3, and the total quantity of WFDC2 protein was calculated.

(3-2) Measurement of First Fragment Quantity

The measurement results in (2-2) were used.

(3-3) Measurement of Second Fragment Quantity

The measurement results in (2-3) were used.

(3-4) Calculation of First Determination Value, Second Determination Value, and Corrected Determination Value

The first fragment quantity obtained from each sample was divided by the total quantity of WFDC2 protein, thereby obtaining the first determination value. Similarly, the second fragment quantity obtained from each sample was divided by the total quantity of WFDC2 protein, thereby obtaining the second determination value. For each sample, a sum of a value obtained by multiplying the first determination value by 10 and the second determination value was obtained as a corrected determination value.

The first determination values, the second determination values, and the corrected determination values obtained above were all normalized in the same manner as in (2-4) with the mean value and the standard deviation of the combined group of the patient group and the healthy individual group. The first determination values, the second determination values, and the corrected determination values normalized in this manner were plotted on the graphs shown in Fig. 5(A), Fig. 5(B), and Fig. 5(C), respectively.

The non-normalized first determination values, the non-normalized second determination values, and the non-normalized corrected determination values were plotted on the graphs shown in Fig. 6(A), Fig. 6(B), and Fig. 6(C), respectively.

(3-5) Determination of Threshold Value

Next, the degree of overlap between the patient group and the healthy individual group was analyzed in each of the normalized first determination values, second determination values, and corrected determination values. Here, the definition of the "degree of overlap" is same as that in (2-5).

First, the difference in the degrees of overlap in Fig. 5(A) and Fig. 5(B), which are cases in which the normalization is performed, is not clearly observed at first glance. The difference in the degrees of overlap in the first determination values in Fig. 5(A) and the corrected determination values in Fig. 5(C) and the difference in the degrees of overlap in the second determination values in Fig. 5(B) and the corrected determination values in Fig. 5(C) are also not clear.

In a case in which the normalization is not performed, the difference in the degrees of overlap in Fig. 6(A) and Fig. 6(B) is not clearly observed at first glance. The difference in the degrees of overlap in the first determination values in Fig. 6(A) and the corrected determination values in Fig. 6(C) and the difference in the degrees of overlap in the second determination values in Fig. 6(B) and the corrected determination values in Fig. 6(C) are also not clear.

Next, the values of ROC-AUC were calculated in a case in which the first determination values, the second determination values, the corrected determination values were used as the indicators for the examination, and the calculated ROC-AUC values were 0.76, 0.72, and 0.76, respectively. From the ROC-AUC values, it is possible to say that the first determination values that are based on the W10409 fragment quantity are slightly superior as the indicators for the examination of lung adenocarcinoma to the second determination values that are based on the W14309 fragment quantity. It is also possible to say that, from the ROC-AUC values, the corrected determination values obtained by combining the first determination values and the second determination values are equal to the first determination values. The ROC-AUC values in a case in which the normalization is not performed are the same as above.

A graph obtained by two-dimensionally plotting the non-normalized second determination values against the non-normalized first determination values for each sample is shown in Fig. 4. In the graph, the circular symbols represent the samples of the healthy individuals, and the square symbols represent the samples of the patients. As can be understood from the graph, no conspicuous values are observed in any of the first determination values and the second determination values for the samples of the healthy individuals, whereas the samples of the patients may show conspicuous values in either the first determination values or the second determination values. In other words, it is assumed that either a mutation that results in the generation of the W10409 fragment or a mutation that results in the generation of the W14309 fragment occurs, in a case in which a mutation occurs in the WFDC2 protein in the lung adenocarcinoma patients. In addition, the frequency of the mutation that results in the generation of the W10409 fragment is higher than the frequency of the mutation that results in the generation of the W14309 fragment, and thus, it is assumed that the first determination values are somewhat superior to the second determination values as the indicators for the examination.

The calculation of the threshold values for the degrees of overlap between the patient group and the healthy individual group was performed in the same manner as in (2-5), by using each of the normalized data shown in Figs. 5(A) to (C) and the non-normalized data shown in Figs. 6(A) to (C), and using MSE as the index and each of the first determination values, the second determination values, and the corrected determination values as the indicators for the examination. The value of t that minimized MSE was designated as the threshold value for each of the determination values.

As a result, MSE for the normalized first determination values was 0.0713, which was the minimum, when the threshold value was -0.31. MSE for the normalized second determination values was 0.047, which was the minimum, when the threshold value was -0.31. MSE for the normalized corrected determination values was 0.0581, which was the minimum, when the threshold value was -0.34.

MSE for the non-normalized first determination values was 0.0004, which was the minimum, when the threshold value was 0.051. MSE for the non-normalized second determination values was 0.004, which was the minimum, when the threshold value was 0.157. MSE for the non-normalized corrected determination values was 0.045, which was the minimum, when the threshold value was 0.68.

ROC-AUC, the threshold value, and MSE for each of the normalized determination values described above are indicated in the following Table 3, and ROC-AUC, the threshold value, and MSE for each of the non-normalized determination values described above are indicated in the following Table 4.

[Table 3]

| Determination value | Fragment | POC-AUC | Threshold value | MSE |
|---|---|---|---|---|
| First determination value | W10409 | 0.76 | -0.31 | 0.0713 |
| Second determination value | W14309 | 0.72 | -0.31 | 0.047 |
| Corrected determination value | W10409 + W14309 | 0.76 | -0.34 | 0.0581 |

[Table 4]

| Determination value | Fragment | POC-AUC | Threshold value | MSE |
|---|---|---|---|---|
| First determination value | W10409 | 0.76 | 0.051 | 0.0004 |
| Second determination value | W14309 | 0.72 | 0.157 | 0.004 |
| Corrected determination value | W10409 + W14309 | 0.76 | 0.68 | 0.045 |

From the above, it is found that ROC-AUC is higher in the determination model using the first determination values based on the detection of the W10409 fragment than in the determination model using the second determination values based on the detection of the W14309 fragment, and thus, the former is superior as the lung adenocarcinoma detection method.

In the determination model using the corrected determination values, in which the second determination values are taken into consideration as well while placing more weight on the first determination values, the ROC-AUC value was equal to that in the determination model using the first determination values alone, whereas MSE was decreased. Since ROC-AUC per se has a certain level of height, it is found to be useful as a determination model for lung adenocarcinoma.

It was also found that each of the determination values may or may not be normalized.

(3-6) Detection of Lung Adenocarcinoma

In a case in which the detection of the W10409 fragment is used alone as the indicator, the lung adenocarcinoma detection method is as follows. That is, urine is collected from a subject who has not been identified as a lung adenocarcinoma patient or a healthy subject yet, and using the urine as a sample, the creatinine concentration in the urine is measured according to (3-1), and the first fragment quantity is measured according to (3-2). The first determination value is calculated and subjected to normalization according to (3-4), and in a case in which the normalized first determination value is -0.31 indicated in Table 3 or higher, the subject can be determined to be positive for lung adenocarcinoma. In the case of not performing the normalization, the subject can be determined positive for lung adenocarcinoma when the first determination value before the normalization is 0.051 indicated in Table 4 or higher.

In a case in which the detection of the W14309 fragment is also used in addition to the detection of the W10409 fragment, the lung adenocarcinoma detection method is as follows. That is, urine is collected from a subject who has not been identified as a lung adenocarcinoma patient or a healthy subject yet, and using the urine as a sample, the creatinine concentration in the urine is measured according to (3-1), the first fragment quantity is measured according to (3-2), and the second fragment quantity is measured according to (3-3). The first determination value and the second determination value are calculated, and the corrected determination value is calculated from these determination values and subjected to normalization according to (3-4). In a case in which the normalized corrected determination value is -0.34 indicated in Table 3 or higher, the subject can be determined to be positive for lung adenocarcinoma. In the case of not performing the normalization, the subject can be determined to be positive for lung adenocarcinoma in a case in which the corrected determination value before the normalization is 0.68 indicated in Table 4 or higher.

(4) Examination of Reference Quantity

In (2) and (3), it was shown that the model using the creatinine concentration as the reference quantity was superior as the lung adenocarcinoma detection method to the model using the total quantity of WFDC2 protein as the reference quantity, even though the models were based on the same first fragment quantity and second fragment quantity.

(5) Example 3

In Example 3, it was verified whether the detection of lung adenocarcinoma was possible using the threshold value of 2.1 of the corrected determination values (10-fold) derived in Example 1.

(5-1) Sample Collection

Among patients showing a nodule shadow or a mass shadow in the lung which is suspected to be primary lung cancer in chest imaging examination, two patients who were diagnosed with lung adenocarcinoma by surgery, transbronchial biopsy, lymph node biopsy, or cytodiagnosis were newly selected. Using sterile cups, first-catch urine was collected from the two selected lung adenocarcinoma patients and ten healthy individuals as samples, and the samples were stored at -80°C until measurement. When performing the measurement, the frozen urine was naturally thawed at room temperature to be used as samples, and the creatinine concentrations in the samples were provided for analysis.

(5-2) Measurement of First Fragment Quantity

The first fragment quantity in each sample was obtained in the same manner as in (2-2), except that the newly collected samples were used in (5-1).

(5-3) Measurement of Second Fragment Quantity

The second fragment quantity in each sample was obtained in the same manner as in (2-3), except that newly collected samples were used in (5-1).

(5-4) Calculation of Corrected Determination Value

Non-normalized corrected determination values (10-fold) were obtained in the same manner as in (2-4) from the first fragment quantities and the second fragment quantities obtained in (5-2) and (5-3).

(5-5) Determination

The presence or absence of cancer was determined based on whether or not each of the non-normalized corrected determination values (10-fold) exceeded the threshold value of 2.1 of the corrected determination values (10-fold) derived in Example 1. In other words, in a case in which a corrected determination value exceeding the threshold value of 2.1 was obtained, it was determined that the individual who provided the sample had lung adenocarcinoma, and in a case in which a corrected determination value below the threshold value of 2.1 was obtained, it was determined that the individual who provided the sample did not have lung adenocarcinoma. As a result, two subjects were determined to have lung adenocarcinoma, and ten subjects were determined to be without lung adenocarcinoma. The two subjects who were determined to have lung adenocarcinoma were the patients who were diagnosed with lung adenocarcinoma by surgery, transbronchial biopsy, lymph node biopsy, or cytodiagnosis. The ten subjects who were determined to be without lung adenocarcinoma were the healthy individuals. The results are shown in Fig. 8.

From the results, it was found that the presence or absence of lung adenocarcinoma can be determined using each of the determination values.

**Claims**

1. An adenocarcinoma detection method based on a protein fragment of WFDC2 protein in a sample originating from a subject, the method comprising:
determining a presence of adenocarcinoma by comparing a first determination value and a threshold value set in advance, the first determination value being a value derived by dividing a first fragment quantity, which is a quantity of a protein fragment having an amino acid sequence of SEQ ID NO: 1 in the sample as determined by a sandwich method, by a reference quantity defined by a total quantity of WFDC2 protein or a creatinine concentration in the sample as determined by a sandwich method.

2. The adenocarcinoma detection method according to claim 1, wherein a quantity of a protein fragment measured using a first anti-C-terminus antibody, which specifically binds to a C-terminal region of the protein fragment having the amino acid sequence of SEQ ID NO: 1, and an anti-N-terminal side region antibody, which recognizes a region nearer to an N-terminal side from the C-terminal region of the protein fragment having the amino acid sequence of SEQ ID NO: 1, is used as the first fragment quantity in the sample.

3. The adenocarcinoma detection method according to claim 1 or 2, wherein a corrected determination value is used instead of the first determination value, the corrected determination value being a sum of a second determination value, which is a value derived by dividing a second fragment quantity that is the quantity of a protein fragment having an amino acid sequence of SEQ ID NO: 2 in the sample as determined by a sandwich method by the reference quantity, and a value obtained by multiplying the first determination value by from 5 to 100.

4. The adenocarcinoma detection method according to claim 3 depending from claim 2, wherein a quantity of a protein fragment measured using a second anti-C-terminus antibody, which specifically binds to a C-terminal region of the protein fragment having the amino acid sequence of SEQ ID NO: 2, and an anti-N-terminal side region antibody, which recognizes a region nearer to to an N-terminal side from the C-terminal region of the protein fragment having the amino acid sequence of SEQ ID NO: 2, is used as the second fragment quantity in the sample.

5. The adenocarcinoma detection method according to claim 2 or claim 4, wherein a quantity of a protein fragment measured using a third anti-C-terminus antibody, which specifically binds to a C-terminal region of a protein fragment having an amino acid sequence of SEQ ID NO: 3, and an anti-N-terminal side region antibody, which recognizes a region nearer to to an N-terminal side from the C-terminal region of the protein fragment having the amino acid sequence of SEQ ID NO: 3, is used as the total quantity of WFDC2 protein.

6. The adenocarcinoma detection method according to any one of claims 1 to 5, wherein the reference quantity is the total quantity of WFDC2 protein.

7. The adenocarcinoma detection method according to any one of claims 1 to 4, wherein the reference quantity is the creatinine concentration.

8. The adenocarcinoma detection method according to any one of claims 1 to 7, wherein the sample is urine of the subject or a sample originating from the urine.

9. An examination kit for determining a presence of adenocarcinoma by a sandwich method, the examination kit comprising:

   a first microplate; and
   two types of antibodies which specifically bind to a protein fragment having an amino acid sequence of SEQ ID NO: 1,
   wherein one of the two types of antibodies is immobilized on the first microplate.

10. The examination kit according to claim 9, wherein the two types of antibodies are a first anti-C-terminus antibody, which specifically binds to a C-terminal region of the protein fragment having the amino acid sequence of SEQ ID NO: 1, and an anti-N-terminal side region antibody, which recognizes a region nearer to an N-terminal side from the C-terminal region of the protein fragment having the amino acid sequence of SEQ ID NO: 1.

11. The examination kit according to claim 10, further comprising:

a second microplate; and
a second anti-C-terminus antibody, which specifically binds to a C-terminal region of a protein fragment having an amino acid sequence of SEQ ID NO: 2,
wherein one of the second anti-C-terminus antibody or the anti-N-terminal side region antibody is immobilized on the second microplate.

FIG.1

EP 4 169 941 A1

FIG.2

(A)

(B)

(C)

EP 4 169 941 A1

FIG.3

EP 4 169 941 A1

# FIG.4

# FIG.5

EP 4 169 941 A1

FIG.6

(A)

(B)

(C)

EP 4 169 941 A1

FIG.7

□ = PATIENT GROUP

○ = HEALTHY INDIVIDUAL GROUP

FIRST DETERMINATION VALUE

SECOND DETERMINATION VALUE

FIG.8

## INTERNATIONAL SEARCH REPORT

| International application No. |
| --- |
| PCT/JP2021/023578 |

**A. CLASSIFICATION OF SUBJECT MATTER**
Int.Cl. C07K16/18(2006.01)i, G01N33/574(2006.01)i, G01N33/70(2006.01)i
FI: G01N33/574AZNA, G01N33/70, C07K16/18

According to International Patent Classification (IPC) or to both national classification and IPC

**B. FIELDS SEARCHED**

Minimum documentation searched (classification system followed by classification symbols)
Int.Cl. C07K16/18, G01N33/574, G01N33/70

Documentation searched other than minimum documentation to the extent that such documents are included in the fields searched
Published examined utility model applications of Japan      1922-1996
Published unexamined utility model applications of Japan    1971-2021
Registered utility model specifications of Japan            1996-2021
Published registered utility model applications of Japan    1994-2021

Electronic data base consulted during the international search (name of data base and, where practicable, search terms used)
JSTPlus/JMEDPlus/JST7580(JDreamIII),
CAplus/REGISTRY/MEDLINE/EMBASE/BIOSIS(STN)

**C. DOCUMENTS CONSIDERED TO BE RELEVANT**

| Category* | Citation of document, with indication, where appropriate, of the relevant passages | Relevant to claim No. |
| --- | --- | --- |
| X<br>A | WO 2017/142025 A1 (UNIV OF MIYAZAKI) 24 August 2017 (2017-08-24), claims, paragraphs [0014]-[0104] | 1-2, 5-11<br>3-4 |
| A | JP 2019-502916 A (F. HOFFMANN-LA ROCHE AG) 31 January 2019 (2019-01-31), entire text, all drawings | 1-11 |
| A | JP 2010-502229 A (VANDERBILT UNIVERSITY) 28 January 2010 (2010-01-28), entire text, all drawings | 1-11 |
| A | US 2014/0348854 A1 (WOMAN & INFANTS' HOSPITAL OF RHODE ISLAND) 27 November 2014 (2014-11-27), entire text, all drawings | 1-11 |

☐ Further documents are listed in the continuation of Box C.          ☒ See patent family annex.

| | |
| --- | --- |
| *     Special categories of cited documents: | "T"  later document published after the international filing date or priority date and not in conflict with the application but cited to understand the principle or theory underlying the invention |
| "A"  document defining the general state of the art which is not considered to be of particular relevance | |
| "E"  earlier application or patent but published on or after the international filing date | "X"  document of particular relevance; the claimed invention cannot be considered novel or cannot be considered to involve an inventive step when the document is taken alone |
| "L"  document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O"  document referring to an oral disclosure, use, exhibition or other means | |
| "P"  document published prior to the international filing date but later than the priority date claimed | "&"  document member of the same patent family |

| Date of the actual completion of the international search | Date of mailing of the international search report |
| --- | --- |
| 19 July 2021 | 03 August 2021 |

| Name and mailing address of the ISA/ | Authorized officer |
| --- | --- |
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

INTERNATIONAL SEARCH REPORT
Information on patent family members

| | International application No. |
|---|---|
| | PCT/JP2021/023578 |

```
WO 2017/142025 A1  24 August 2017       (Family: none)

JP 2019-502916 A    31 January 2019      US 2018/0292410 A1
                                         entire text, all drawings
                                         WO 2017/103034 A1
                                         EP 3391053 A1
                                         CN 108369233 A
                                         KR 10-2018-0088840 A
                                         BR 112018008623 A2
                                         HK 1259002 A1

JP 2010-502229 A    28 January 2010      US 2008/0057514 A1
                                         entire text, all drawings
                                         WO 2008/030979 A2
                                         EP 2064349 A2
                                         CN 101535506 A

US 2014/0348854 A1 27 November 2014      WO 2012/170513 A2
                                         EP 2717901 A2
                                         CN 104039343 A
```

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017142025 A1

**Non-patent literature cited in the description**

- **SAWABATA, N. et al.** Japanese lung cancer registry study of 11,663 surgical cases in 2004: demographic and prognosis changes over decade. *J. Thorac. Oncol.,* 2011, vol. 6 (7), 1229-1235
- **ATAGI, S. et al.** Thoracic radiotherapy with or without daily low-dose carboplatin in elderly patients with non-small-cell lung cancer: a randamised, controlled, phase 3 trial by the Japan Clinical Oncology Group (JCOG0301). *Lancet Oncol.,* 2012, vol. 13, 671-678
- **SCAGLIOTTI, G.V et al.** Phase III study comparing cisplatin plus gemcitabine with cisplatin plus pemetrexed in chemotherapy-naive patients with advanced-stage non-small-cell lung cancer. *J. Clin. Oncol.,* 2008, vol. 26 (21), 3543-3551
- **MOLINA, R. et al.** Assessment of a Combined Panel of Six Serum Tumor Markers for Lung Cancer. *Am. J. Respir. Crit. Care Med.,* 2016, vol. 193 (4
- **MATSUOKA, K. et al.** Prognostic value of carcinoembryonic antigen and CYFRA21-1 in patients with pathological stage I non-small cell lung cancer. *Eur. J. Cardiothorac. Surg.,* 2007, vol. 32 (3), 435-439
- **OHMI SHINOBU ; KUNIO TSUJIMURA ; MASAKI INAGAKI.** New Edition Anti-Peptide Antibody Experimental Protocol. Gakken Medical Shujunsha Co., Ltd, 06 September 2004